(19) Europäisches Patentamt
European Patent Office
Office européen des brevets

(11) **EP 4 389 711 A1**

(12) **EUROPEAN PATENT APPLICATION**

(43) Date of publication:
**26.06.2024 Bulletin 2024/26**

(21) Application number: **22383286.6**

(22) Date of filing: **23.12.2022**

(51) International Patent Classification (IPC):
*C02F 1/66* (2023.01)   *C02F 1/68* (2023.01)
*C02F 11/00* (2006.01)   *C02F 11/02* (2006.01)
*C02F 103/28* (2006.01)   *C02F 3/34* (2023.01)
*C12P 7/56* (2006.01)   *C12P 7/46* (2006.01)
*C12P 7/625* (2022.01)   *C08J 11/04* (2006.01)
*C13K 1/02* (2006.01)   *C12P 19/14* (2006.01)
*C08H 8/00* (2010.01)

(52) Cooperative Patent Classification (CPC):
**C02F 1/66; C02F 1/683; C02F 11/004; C02F 11/02;
C08J 11/105; C12P 7/18; C12P 7/42; C12P 7/46;
C12P 7/56; C12P 19/02; C12P 19/14;
C12Y 302/01004; C12Y 302/01021; C13K 1/02;**
C02F 3/342;                                    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **INSTITUTO TECNOLÓGICO DEL
EMBALAJE, TRANSPORTE
Y LOGÍSTICA (ITENE)
46980 Paterna (ES)**

(72) Inventors:
• ZABALETA MERI, Javier
  **46980 PATERNA (ES)**
• DÍAZ EXPÓSITO, Licinio
  **46980 PATERNA (ES)**
• GONZÁLEZ BUCH, Cristina
  **46980 PATERNA (ES)**
• CUBAS CANO, Enrique
  **46980 PATERNA (ES)**

(74) Representative: **ZBM Patents - Zea, Barlocci &
Markvardsen
Rambla Catalunya, 123
08008 Barcelona (ES)**

(54) **PROCESS OF DECONTAMINATION OF A RECOVERED CELLULOSIC WASTE STREAM FOR ITS SUBSEQUENT TRANSFORMATION INTO FERMENTABLE SUGARS**

(57) It relates to a process of decontamination of a cellulose recovered waste stream to obtain a decontaminated cellulose stream that can be converted into fermentable sugars. The decontamination process comprises first, submitting the recovered cellulosic waste stream to an alkaline treatment. The process may also comprise submitting the treated cellulose to a saccharification process to obtain a hydrolysate which comprises fermentable sugars. Finally, the invention may also comprise the fermentation process.

FIG. 3

EP 4 389 711 A1

(52) Cooperative Patent Classification (CPC): (Cont.)
C02F 2103/28; C02F 2303/24; C08J 2301/02;
C12P 2201/00; C12P 2203/00

**Description**

**Technical Field**

The invention relates to a process of decontamination of a recovered cellulosic waste stream to obtain a decontaminated cellulose stream, that can be converted into a hydrolysate comprising fermentable sugars.

**Background Art**

Up to 138 million tons of bio-waste, coming from wastewater treatment and urban organic food are annually generated in the EU (EUR-Lex, 2020). It is estimated that almost 75% of this waste is sent to incineration or landfilling, with a huge environmental and economic cost associated. Furthermore, wastewater contains valuable components, such as cellulose and nutrients that can be used as feedstock for many applications. Domestic wastewater is often treated in activated sludge systems, which present high operating costs and energy demand with a low performance and high carbon footprint. Moreover, most of the nutrients and valuable components that could be potentially recovered are dissipated.

Municipal sewage water contains a high number of suspended solids, and up to 70% of this material consists of cellulose fibers originated from the use of toilet paper. This cellulose is a valuable resource, but also puts an additional burden on the wastewater treatment process. The presence of certain pollutants could interfere in several downstream processes and prevent the achievement of the targeted yields in downstream processes, more precisely, cellulose from wastewater may have specific contaminants that can affect the valorization processes, such as saccharification and subsequent fermentation to obtain compounds that can be used as starting point for biopolymers production, such as lactic acid, succinic acid, itaconic acid or 1,4-butanediol. Furthermore, presence of certain pollutants could also affect processes to obtain polyhydroxyalkanoates (PHA), a class of biopolyester which can be produced from holocellulose-derived sugars by aerobic fermentation, intracellular accumulation, and extraction.

Different treatments of cellulose materials have been disclosed in the art. These methods include acid treatment using, for instance $H_2SO_4$ at low concentration, oxidant treatments employing $H_2O_2$, and also alkaline treatments using sodium hydroxide.

Several alkaline treatments for different type of residues have been disclosed in the art. For instance, A. ilbazarri et al.; "Alkali treated foumanant tea waste as an efficient adsorbent for methylene blue adsorption from aqueous solution", Water Resour Ind, 2014, 6, 64-80 describes that alkaline (NaOH) treatments are one of the chemical methods of treatment of agricultural foumanant tea waste to improve adsorption properties; also Y. Zhou et al, "Removal of organic pollutants from aqueous solution using agricultural wastes: a review, Journal of Molecular liquids 2015, 212, 739-762 describes that for agricultural wastes, alkaline treatment is widely used as a chemical treatment to modify these wastes due to their hydrolysis; or CA2071290AA, which describes a method to obtain white cellulose from palm leaves by treating them with 3.7% NaOH solution.

US2002185447A1 indicates that any suitable alkaline or acid catalyst can be used to convert the fiber and hemicellulosic material in the sewage sludge to a carbohydrate. Alkali metal compounds, such as alkali metal oxides, alkali metal hydroxide and mixtures thereof may be used. Mixtures of sodium hydroxide may be used.

CN106914225B uses an alkaline pretreatment with hot NaOH in a process to obtain an adsorbent from waste-water, dye water or cellulose substrates. In Examples it is illustrated from sugar cane, cotton fiber or viscose.

Finally, US2012083542A describes the treatment of domestic, industrial or production waste with an aqueous, alkaline solution at 140-250 °C and a pressure between 3 bar and 12 bar.

Generally, known alkaline treatments are accompanied with further components or treatments, such as for example bleaching treatments. Furthermore, they use concentrated solutions with high temperatures and pressures and produce residuals difficult to eliminate.

On the other hand, methods of biomass fermentation to produce, for instance, L-lactic acid comprising an alkaline pretreatment are known. For instance, CN102174602A discloses a method to produce L-lactic acid by biomass (corn cob) fermentation to which an alkaline pretreatment with NaOH 6%, at 100 °C, for 1 h has been carried out.

From what is disclosed in the prior art, it is derived that there is still the need of providing an efficient, economical, and sustainable decontamination method able to remove the main contaminants present in the cellulose recovered from waste streams, in order to avoid interferences in the subsequent downstream processes (such as saccharification and subsequent fermentation) to obtain useful products from such waste streams.

**Summary of Invention**

Inventors have found that by using an alkaline treatment with specific mild conditions to decontaminate waste streams comprising cellulose, it is possible to transform the decontaminated cellulosic stream into a hydrolysate comprising fermentable sugars, being not only the saccharification step, but also the subsequent fermentation step to obtain lactic acid, succinic acid, itaconic acid, 1,4-butanediol or polyhydroxyalkanoates, more efficient processes, obtaining an increase in performance. Thus, the process of the present invention allows the decontamination of a recovered cellulosic waste stream to be reused (transformation into 2nd generation sugars), with the associated savings in wastewater treatment plants.

Unlike other known processes that generate toxic secondary compounds that interfere with the fermentation processes, the process of the present invention avoids the generation of secondary reactions that could generate new toxic compounds. Moreover, the contaminants that remain in the cellulose treated by the method of the invention do not interfere negatively in its subsequent saccharification and fermentation. Thus, the method of decontamination is efficient, economical, sustainable, and allows to remove the main pollutants present in the cellulose recovered from waste streams, avoiding interferences in the subsequent downstream processes.

Accordingly, the present invention relates to a process of decontamination of a recovered cellulosic waste stream which comprises the following steps: a) submitting the recovered cellulosic waste stream to an alkaline treatment which comprises the following steps: a1) combining the recovered cellulosic waste stream with diethylene triamine pentaacetate (DTPA) and a base to yield an alkaline medium wherein the ratio cellulose:base is in a range from 8:1 to 3:1 weight %; a2) heating up the reaction mixture to a temperature in a range from 75 to 90°C and at atmospheric pressure (1 atm) for at least 1 hour; and a3) separating the decontaminated cellulosic stream.

The process may further comprise a subsequent transformation step of the decontaminated cellulose stream into a hydrolysate comprising fermentable sugars.

**Brief Description of Drawings**

FIG. 1 shows fiber suspension take by the CCD video camera.

FIG. 2 shows the average and distribution for real length and width measurements (according to ISO/FDIS 160652).

FIG. 3 shows the lactic acid concentration in g/L of the following cellulose samples: control for the pure cellulose sample, treatment according to the process of the present invention (Method 2), and the untreated cellulose sample.

FIG. 4 shows the total pollutants present in the non-treated cellulose and the pollutants presents in the decontaminated cellulosic stream with the method of the present invention.

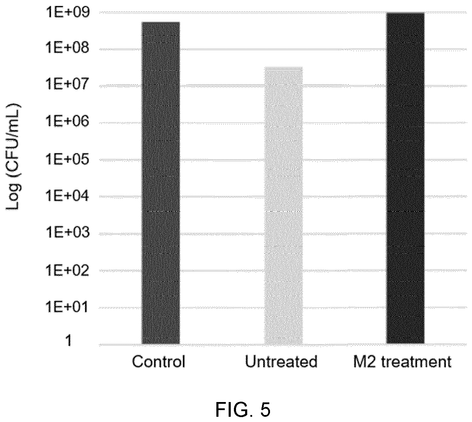

FIG. 5 shows a logarithm of the colony-forming unit per milliliter. The control is pure cellulose, the untreated samples without any decontamination process, Comparative treatment (method 1) and treatment of the present invention (method 2). These *Pediococcus pentosaceus* colonies were counted after 48 h of incubation at 30 °C in MRS medium.

**Detailed description of the invention**

All terms as used herein in this application, unless otherwise stated, shall be understood in their ordinary meaning as known in the art. Other more specific definitions for certain terms as used in the present application are as set forth below and are intended to apply throughout the description and claims

Where in the present invention a numerical interval is used, this includes the values of the extremes of the interval.

The word "comprise" for the purposes of the present invention encompasses the case of "consisting of".

The term "atmospheric pressure" means the standard atmospheric pressure at any altitude and under any weather conditions at the location whether the process is conducted. The standard atmospheric pressure at sea level is approximately 1 atm (1013 millibars). Variations about these values are quite small, for instance, atmospheric pressure decreases with increasing elevation.

As used herein, the term "cellulose or cellulosic" in the present invention refers to cellulose that mainly is "holocellulose".

As mentioned above, the process of decontamination of a recovered cellulosic waste stream comprises the following steps: a) submitting the recovered cellulosic waste stream to an alkaline treatment which comprises the following steps: a1) combining the recovered cellulosic waste stream with diethylene triamine pentaacetate (DTPA) and a base to yield an alkaline medium wherein the ratio cellulose:base is in a range from 8:1 to 3:1 weight %; a2) heating up the reaction mixture at a temperature in a range from 75 to 90°C and at atmospheric pressure for at least 1 hour; and, a3) separating the decontaminated cellulosic stream. A decontamination higher than 95%, can be obtained in step a3) as determined by the UPLC-TOF technique.

It is part of the present invention a process of decontamination of a recovered cellulosic waste stream and subsequent transformation of the decontaminated cellulose stream into a hydrolysate comprising fermentable sugars, the process comprises the following steps: first carrying out the decontamination process as described above, and subsequently, carrying out a further step b) which comprises submitting the decontaminated cellulosic stream from step a3) to a saccharification process to obtain a hydrolysate which comprises fermentable sugars. The saccharification process comprises a hydrolysis of the decontaminated cellulosic stream into fermentable sugars by a cocktail of enzymes.

Finally, it is also considered part of the present invention a process of decontamination of a recovered cellulosic waste stream, subsequent transformation of the decontaminated cellulose stream into a hydrolysate comprising fermentable sugars, and subsequent fermentation to obtain lactic acid, succinic acid, itaconic acid, 1,4-butanediol, or polyhydroxyalkanoates, the process comprises first carrying out the decontamination process as described above as well as the subsequent saccharification as described above to obtain a hydrolysate comprising fermentable sugars, followed by submitting the hydrolysate which comprises fermentable sugars to a fermentation process in the presence of a microorganism capable of producing a compound selected from the group consisting of lactic acid, succinic acid, itaconic acid 1,4-butanediol, and polyhydroxyalkanoates, at an appropriate temperature, and an appropriate incubation time.

The recovered cellulosic waste stream used as initial waste stream in the process of the present invention comes from a waste stream that arrives to the waste-water treatment plant and which undergoes filtration to get a stream that is rich in cellulose named herein recovered cellulosic waste stream. The recovery of cellulose from wastewater is based on the separation of toilet paper cellulose fibres from wastewater. It can be performed by using fine screening technology. For instance, using a fine sieve (or finescreen), cellulosic screenings from sewage water are separated. Examples of fine-sieves or finescreens are those provided by companies like Salsnes. The stream that is rich in cellulose obtained from the initial filtration process can be treated through the decontamination process according to the present invention.

The initial waste stream can be selected from the group consisting of sewage sludge, municipal sewage water, and domestic wastewater. In a particular embodiment, the waste stream is a waste stream of domestic origin. Thus, the recovered cellulosic waste stream used as starting material of the process of the present invention is obtainable from a waste stream as disclosed above.

In a particular embodiment of the process of the present invention, the recovered cellulosic waste stream has a moisture content below 20% by weight. In another particular embodiment, the recovered cellulosic waste stream has a moisture content below 15% by weight. In another particular embodiment, the recovered cellulosic waste stream has a moisture content below 10% by weight.

The recovered cellulosic waste stream may comprise cellulose, hemicellulose, and lignin. In a particular embodiment of the process of the present invention, the recovered cellulosic waste stream comprises holocellulose in an amount comprised in the range from 80% to 90% by weight with respect to the recovered cellulosic waste stream. Both the input stream (the recovered cellulosic waste stream) and the decontaminated cellulosic stream are rich in holocellulose.

Holocellulose is defined as the water-insoluble carbohydrate fraction present in plant raw materials. Therefore, it joins the cellulose fractions and the hemicellulose present in the fibers. It corresponds to the sum of $\alpha$-cellulose and hemicellulose. Both are decontaminated and transformed into sugars in the hydrolysis process.

Among the pollutants present in the recovered cellulosic waste stream from a waste stream there are medicines, laundry detergents, surfactants, dyes, paints, preservatives, food additives, volatile organic compounds (VOC), among others. The presence of specific contaminants could interfere in several downstream processes and prevent the achievement of the targeted yields in downstream processes. In particular, the recovered cellulosic waste stream comprises pollutants selected from the group consisting of phenolic compounds, amides, organic acids, aromatic compounds, and fatty acids. These pollutants may be identified and quantified using UPLC-TOF and GC-TOF techniques. Among the identified compounds with most significant levels in the recovered cellulosic waste stream there are: hexamethylene diisocyanate, hexadecanamide, N-tetradecanamide, and oleamide usually found in the cosmetic sector. Moreover, there are some food additives, such as ascorbic acid, stearic acid, oleic acid, palmitic acid, and catechin hydrate.

The recovered cellulosic waste stream may comprise $\alpha$-cellulose in an amount comprised in the range from 70% to 95% by weight with respect to hollocellulose. In a particular embodiment, the recovered cellulosic waste stream comprises $\alpha$-cellulose in an amount comprised in the range from 75% to 90% by weight with respect to hollocellulose. In another particular embodiment, the recovered cellulosic waste stream comprises $\alpha$-cellulose in an amount comprised in the range from 80% to 90% by weight with respect to hollocellulose. In another particular embodiment, the recovered cellulosic waste stream comprises $\alpha$-cellulose in an amount comprised in the range from 75% to 80% by weight with respect to hollocelulose. In another particular embodiment of the process, the recovered cellulosic waste stream comprises hemicellulose in an amount comprised in the range from 5% to 15% by weight with respect to holocellulose. In another particular embodiment of the process, the recovered cellulosic waste stream comprises hemicellulose in an amount comprised in the range from 6% to 10% by weight with respect to hollocellulose. In another particular embodiment of the process, the recovered cellulosic waste stream comprises α-cellulose in an amount comprised in the range from 75% to 90% by weight with respect to hollocellulose; and hemicellulose in an amount comprised in the range from 5% to 15% by weight with respect to hollocellulose.

In a particular embodiment of the process, the recovered cellulosic waste stream is in the form of fibers which have a length comprised in a range from 800 to 1100 μm. In another particular embodiment of the process, the recovered cellulosic waste stream is in the form of fibers which have a length comprised in a range from 900 to 1000 μm. In another particular embodiment of the process, the recovered cellulosic waste stream is in the form of fibers which have a length comprised in a range from 950 to 1000 μm. In another particular embodiment of the process, the recovered cellulosic waste stream is in the form of fibers which have a width comprised in a range from 10 to 25 μm. In another particular embodiment of the process, the recovered cellulosic waste stream is in the form of fibers which have a width comprised in a range from 1 to 25 μm. In another particular embodiment of the process, the recovered cellulosic waste stream is in the form of fibers which have a length comprised in a range from 800 to 1100 μm; and a width comprised in a range from 10 to 25 μm. The length and width of the cellulose fibers are measured using a MorFi fiber analyzer following the method disclosed in the examples section.

The process of decontamination and separation of cellulose of the present invention is based on treating the recovered cellulosic waste stream coming from wastewater with a high content of cellulose fibers by means of an alkaline treatment, carried out at a certain temperature for a specific time and with certain base concentrations. The alkaline treatment of the present invention modifies the surface of the fibers by removing a certain rate of lignin, hemicellulose, wax, and oils covering the external surface. During the alkaline treatment, the fibers are separated from one another, resulting in an increase in the effective surface area available for wetting. In addition, the alkaline treatment modifies the crystallinity, the unit cell structure, and fiber orientation. Without being bound to any theory, it is believed that the combination of the pH of the treatment with the presence of the chelating agent diethylenetriamine pentaacetate (DTPA) facilitates the extraction and elimination of the pollutants and the removal of the heavy metals, while using milder conditions.

In a particular embodiment, the ratio cellulose:base in the alkaline treatment is comprised in a range from 8:1 to 3:1 weight %. In another particular embodiment, the ratio cellulose:base in the alkaline treatment is comprised in a range from 7:1 to 4:1 weight %. In another particular embodiment, the ratio cellulose:base in the alkaline treatment is comprised in a range from 6:1 to 4:1 weight %. In a particular embodiment, the ratio cellulose:base in the alkaline treatment is comprised in a range from 6:1 to 5:1 weight %. In another particular embodiment, the ratio cellulose:base is 5:1 weight %. In another particular embodiment the base is sodium hydroxide.

In a particular embodiment, the alkaline treatment of the process of decontamination of a recovered cellulosic waste stream according to the invention comprises using in the step a1) a base in the form of an alkaline aqueous solution.

The base in step a1) provides an alkaline medium comprising the recovered cellulose waste stream to decontaminate. In a particular embodiment of the process, the alkaline medium in the alkaline treatment has a concentration of base in a range comprised from 1 to 5% by weight. In another particular embodiment of the process, the alkaline medium has an amount of base in a range comprised from 1 to 4% by weight. In another particular embodiment of the process, the alkaline medium has an amount of base in a range comprised from 1 to 3% by weight. In another particular embodiment of the process, the alkaline medium has an amount of base in a range comprised from 1 to 2% by weight. In another particular embodiment of the process, the base in any of the embodiments above is sodium hydroxide.

In a particular embodiment of the process, the alkaline medium in the alkaline treatment has an amount of diethylenetriamine pentaacetate (DTPA) in a range comprised from 0.1 to 1 %w/w. In another particular embodiment of the process, the alkaline medium in the alkaline treatment has an amount of the diethylenetriamine pentaacetate in a range comprised from 0.3 to 0.75 %w/w. In yet another particular embodiment of the process, the alkaline medium in the alkaline treatment has an amount of diethylenetriamine pentaacetate in an amount of 0.3 %w/w.

In another particular embodiment of the process, the temperature of the alkaline treatment is comprised between 80-85 °C. In another particular embodiment of the process, the alkaline treatment lasts for at least 1 hour. In another particular embodiment of the process, the alkaline treatment lasts for at least 2 hours.

In a particular embodiment, the alkaline treatment carried in the decontamination process comprises the following conditions:1%w/w NaOH in the alkaline medium with diethylenetriamine pentaacetate in an amount of 0.3%w/w, at a temperature of 85°C and atmospheric pressure for 1-2 hours. As it is illustrated in the Examples, the decontamination was greater than 95%, as measured by the UPLC-TOF technique. Thus, most of the contaminants were removed from the cellulose fibers. Data indicates that there was a reduction in most of the volatile contaminants, representing a value of less than 10% of the original sample. A high effectiveness in removing heavy metals was also observed (72.5%).

In a particular embodiment, the amount of cellulose (mainly holocellulose) in the alkaline medium of step a) of the process of the present invention is comprised in a range from 1 to 25% by weight. In another particular embodiment, the amount of cellulose is in a range from 2 to 15% by weight. In another particular embodiment, the amount of cellulose is in a range from 4 to 10% by weight.

After carrying out the alkaline treatment, the solid components of the waste are separated from the aqueous phase. These solid components once separated are the so-called decontaminated cellulosic stream. Thus, in another particular embodiment of the process, the separation of the cellulose from the alkaline solution is done by filtration and/or decantation. The decontaminated cellulosic stream may be washed with water once or several times. Generally, the treated cellulose has a high moisture content (e.g., 70%w/w). Generally, the decontaminated cellulosic stream comprises holocellulose in an amount comprised in the range from 80% to 90% by weight.

Unlike the known alkaline treatments of cellulose fibers from wastewater, the process of the present invention does not carry out a bleaching step together or subsequently to the alkaline treatment. In a particular embodiment of the process, the alkaline treatment is carried out in the absence of an oxidizing agent.

The efficiency of the method was confirmed when carrying out the saccharification process and subsequent fermentation of the previously decontaminated cellulosic stream. Glucose is the main product of the cellulose hydrolysis, and lactic acid is the main product of the fermentation process. The results showed an improvement in cellulose hydrolysis after treating the recovered cellulosic waste stream with the alkaline treatment according to the invention. The still present contaminants in the decontaminated cellulosic stream did not significantly affect the process. No negative effect on fermentation was observed and it behaved similarly to the control.

Thus, in a particular embodiment of the process of the present invention, the subsequent saccharification process (hydrolysis of the decontaminated cellulosic stream of step a3) into fermentable sugars) is carried out by a consortium of enzymes. Different examples of commercial enzymatic cocktails can be used for conducting saccharification step of the process of the present invention, including: β-glucosidases (600-1000 U/g) (Enzymatic cocktail 1); cellulases (100-200 U/g), xylanases (100-200 U/g), and β-glucosidases (300-600 U/g) (Enzymatic cocktail 2); and endo and exo cellulases (100-200 U/g) (Enzymatic cocktail 3).The high saccharification yields are due to the presence of a consortium of different hydrolytic enzymes, able to hydrolyze the β (1-4) glycosidic linkage between the glucose units of the main chains, such as the β (1-6) branches.

In a particular embodiment, the enzyme concentrations may be equal to or higher than 0.05 g or ml/ g of dried solid. In a particular embodiment, the enzyme concentration may be in a range from 0.05 to 0.15 ml/g dry solid.

The percentage of solid in the hydrolysis process may be around 4-20%. In another particular embodiment, the percentage of solid in the hydrolysis process is 8-16%. In another particular embodiment, the percentage of solid in the hydrolysis process is 12%.

In a particular embodiment, the temperature of the saccharification step is comprised in a range from 45 to 50 °C. In another particular embodiment, the temperature for Enzymatic cocktail 1 is preferably 40 °C while the temperature when Enzymatic cocktail 2 or Enzymatic cocktail 3 are used is preferably 50 °C. In another particular embodiment the pH is comprised in a range from 4 to 5. In another particular embodiment, the pH is 4.5. In another particular embodiment, the agitation is comprised in a range from 90 to 300 rpm. In another particular embodiment of the process, the time is at least 24 hours. In another particular embodiment of the process, the time is in a range from 24 to 48 hours. In another particular embodiment of the process, the hydrolysis conditions are 24h-48 h at 45°C and with stirring at 150 rpm.

In another particular embodiment, the medium where the hydrolysis takes place comprises an antioxidant, sodium potassium tartrate Rochelle salt (sodium-potassium tartrate, and sodium hydroxide.

The deactivation of the enzyme and the sterilization of the reaction medium may be carried out by heating in the range from 80 to 90 °C.

As illustrated in the Examples, the decontamination process of the present invention not only does not affect to subsequent saccharification step but improves the glucose yield regarding to the control.

The hydrolysate obtained in the saccharification process has high sugars concentration and can be employed by microorganisms as culture broth. Thus, a fermentation process may be further carried out to produce lactic acid, succinic acid, itaconic acid, 1,4-butanediol, or polyhydroxyalkanoates from the fermentable sugars obtained in the saccharification process. These compounds can be widely used in food, medicine, agriculture and chemical industries. The microbial fermentation for production of these compounds from the fermentable sugars obtained by the process of the present invention is advantageous because raw materials are cheap, the production process is clean, and the production efficiency is high.

There is a great spectrum of microorganisms able to convert different sugars derived from cellulose and hemicellulose into bio-based products that can act as monomers for biopolymers. Different organic acids with applications as monomers for biopolymers production can be produced by microbial fermentation.

Lactic acid is one of the most demanding building blocks for polylactic acid (PLA) which is a common bioplastic currently in the market. Lactic acid is produced by fermentation of different sugars using lactic acid bacteria and some sporulating bacteria. For the production of lactic acid, the fermentation may be carried out at room temperature, in the presence for instance, of *Pediococcus pentosaceus* and incubating at a temperature in a range from 25 to 35 °C, preferably, 30 °C during the appropriate period of time, such as for at least 70 hours, for instance, for 70-75 hours. Other lactic bacteria may also be used.

As it is also illustrated in the Examples, when a hydrolysate obtained from the process of the present invention comprising the decontamination of a recovered cellulosic waste stream and saccharification into a fermentable sugar is then used in a fermentation process, it does not negatively affect to the lactic acid production, and to the viability of strain *P. pentosaceus.*

The microorganisms used are commercially available and can be prepared following the protocols provided by the supplier of the commercial product. They are available from ATCC (hftps://www.lgcstandards-atcc.org/en/About/About_ATCC/Who_We_Are.aspx).

Succinic acid can also be produced by fermentation of released sugars, being potentially used as monomer for the production of polybutylene succinate (PBS) by direct esterification with 1,4-butanediol.

1,4-butanediol is also an interesting monomer for bioplastics production, like PBS. PBS can be prepared by reacting 1,4-butanediol with succinic acid. 1,4-butanediol can be produced by fermentation as a more sustainable option than using fossil feedstocks and energy-intensive processes (J. Cheng et al., "Achievements and Perspectives in 1,4-Butanediol Production from Engineered Microoganisms", 2021, J. Agric. Food Chem, 69, 10480-10485).

Itaconic acid can also be bio produced from holocellulose-derived sugars using microorganisms, being a promising monomer for the production of a wide range of polymers such as methyl methacrylate, polyester resins or superabsorbent polymers. Several microorganisms can be used as microbial cell factories in itaconic acid production such as *A. terreus,* and *U maydis.* (See. D. Gopaliya et al, "Recent advances in itaconic acid production from microbial cell factories", Biocatalysis and Agricultura Biotechnology. 2021, vol. 36, 102130, pp.1-14).

Furthermore, polyhydroxyalkanoates (PHA), a class of biopolyester can also be produced from holocellulose-derived sugars by aerobic fermentation, intracellular accumulation, and extraction (see R. Reshmy et al; "Bioplastic production from renewable lignocellulosic feedstrocks; a review; Rev, Environ. Sic. Biotechnol, 2021, vol. 20, pp. 167-187).

Finally, the decontamination process of a recovered cellulosic waste stream and the subsequent transformation into fermentable sugars of the present invention may be carried out, in batch form, semi batch or continuous form. In a particular embodiment, the process is carried out in semi batch form.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

**Examples**

Example 1: Cellulose characterization:

Cellulose characterization was carried out according to TAPPI (Technical Association of the Pulp and Paper Industry) standards, test procedures and analyses related to a series of ISO and ASTM standards that are used in the measurement, evaluation and characterization of pulp, paper, and related products, including the raw materials used in their manufacture.

These standards and guidelines have been developed and approved by the Quality and Standards Management Committee with the advice and consent of TAPPI's Board of Directors. For the chemical characterization of the sample, the procedures and standards of the T200 series "Fibrous materials and pulp testing" listed below were followed:

- Determination of moisture content (TAPPI T264 cm-97).
- Determination of holocellulose (Wise et al. 1946).
- Determination of $\alpha$-cellulose and hemicellulose (Rowell et al. 1982).

Moisture content analysis: To determine the moisture content of cellulose samples, 2 g of the sample ($m_0$) were weighed in a moisture-free crucible and previously tared scale (mc). Subsequently, the whole sample was subjected to an oven drying process at 105 °C for 24 hours. After cooling in a desiccator, the crucible was weighed together with the dry residue ($m_1$), determining the moisture of the sample according to the following equation:

$$\%\text{moisture} = \frac{m0 - (m1 - mc)}{m0} \times 100$$

Total content in cellulose, hemicellulose, and lignin:

Holocellulose determination by Wise method (see L.E. Wise et al., Tech. Assoc. Papers 1946, vol. 29, p.210). The method proposed by Wise et al. (1946) is based on the fact that the chlorine dioxide released in successive treatments with sodium chlorite dissolves the lignin, while the carbohydrates remain unchanged.

To carry out this method, 2.5 g of sample free of extracts and moisture ($m_0$) was introduced into an Erlenmeyer flask and 80 mL of hot distilled water was added (70-80 °C). The mixture was heated to 70 °C and then 0.5 mL of glacial acetic acid and 2.6 mL of 25% sodium chlorite were added (every hour) to cover a total of 6-8 h time period. The mixture was then heated up to 70 °C and 0.5 mL of glacial acetic acid and 2.6 mL of 25% sodium chlorite were added (every hour) to cover a total of 6-8 h time period. After some time, the mixture remained covered in the bath for a further 12 hours without further input. The suspension was then vacuum filtered using a porous plate from N° 2 (previously tared after 24 hours at 105 ± 3 °C, ($m_f$)) and washed with hot distilled water, determining the % of holocellulose by the following expression.

$$\%\text{holocellulose} = \frac{(m1 - mf)}{m0} \times 100$$

$\alpha$-cellulose and hemicellulose by Rowel's method (see R.M. Rowell, "Distribution of acetyl groups in southern pine reacted with acetic anhydride" Wood Sic, vol. 15, pp.172-182). The term 'hemicellulose' refers to components of the plant cell wall which are solubilized by treatment with sodium hydroxide and acetic acid, whereas $\alpha$-cellulose corresponds to the fraction of holocellulose which remains insoluble after such a treatment. According to the method used, 2 g of dry holocellulose sample free of extracts ($m_0$) were weighed and 10 mL of NaOH were added to 17.5% by weight, stirring until a correct dispersion was achieved. Subsequently, 5mL of 17.5% NaOH was added every 5 minutes until a total of 25 mL was reached (3 additions of 5 mL). The suspension was then left to react for 30 minutes at 20°C and then 33 mL of distilled water was added to 20 °C, leaving 1 hour to stand. The resulting mixture was vacuum filtered on a porous plate of N°1 (previously tared after 24 hours at 105 ± 3 °C, (mf)). The residue was washed with 100 mL of an 8.3% NaOH solution by weight, after which it was washed with distilled water twice more. Subsequently, 15 mL of 10% acetic acid was added, allowing it to react 3 minutes before switching on the vacuum. It was finally washed with distilled water until the filtrate is free of acid (neutral pH). The filter/residue was dried at 105 °C and cooled down in a desiccator ($m_1$), determining the content of $\alpha$-cellulose in the initial holocellulose sample by the following expression:

$$\%\alpha\text{-cellulose (H)} = \frac{(m1 - mf)}{m0} \times 100$$

The real percentage of $\alpha$-cellulose in the fiber was determined as follows:

$$\%\alpha\text{-cellulose} = \frac{(\%\alpha\text{-cellulose} \times \%\text{holocellulose})}{100}$$

Finally, the hemicellulose content was calculated by difference:

$$\%\text{hemicellulose} = \%\text{holocellulose} - \%\alpha\text{-cellulose}$$

Cellulose fiber analysis (average length and width): The fiber morphology of the sample supplied by AQUALIA was analyzed using the MorFi fiber analyzer. 25 g of the cellulose sample (dried) was diluted in 1000 mL of distilled water. The resulting suspension was introduced in the equipment and the fiber length and width were assessed by image analysis in a transparent channel equipped by a CCD video camera, analyzing 10,000 fibers/analysis.

Sample used (Sample 1): recovered cellulosic waste stream sample obtained after applying a fine screen separation process to the initial waste stream of a domestic wastewater treatment plant, provided by AQUALIA.

Results: Sample 1 had a moisture content of 9.4 ± 0.4%, after triplicate analysis of a homogeneous sample.

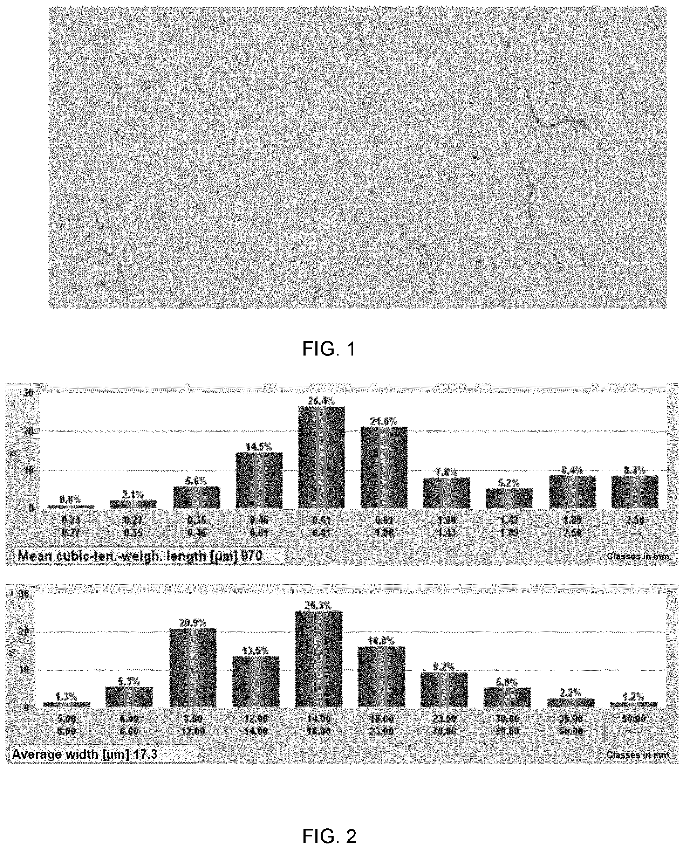

Regarding the calculation of holocellulose content, the average percentage was 85.89%, represented by 76.6% $\alpha$-cellulose and 9.25% hemicellulose. After characterization of the morphology in terms of length and width, they showed an approximate size of 970 $\mu$m and 17.3 $\mu$m, respectively. FIG. 1 shows the fiber suspension take by the CCD video camera.

FIG. 2 shows the average and distribution for real length and width measurements (according to ISO/FDIS 160652). These parameters show an ideal size for the development of the consequent valorization processes, as well as a moisture content that avoids microbial growth and the correct preservation of the residue. The high cellulose content in the sample 1, is indicative of the high recovery potential of this residue since more than 85% of the biomass is made up of holocellulose, representing almost 80% of $\alpha$-cellulose. This indicates that 80% of the material is potential to be subjected to the saccharification process and be transformed into 2nd generation sugars.

Example 2: Identification of main pollutants present in sample 1.

Extraction protocol and analysis by UPLC-TOF and GC-TOF: First, differential extraction was performed on solvents of different polarities, such as water, ethanol, acetone, and hexane so that as many contaminants as possible are extracted from the recovered cellulose. Subsequently, the extracts obtained were analyzed by UPLC-TOF and GC-TOF. Once the identification was carried out, the efficiency of the decontamination processes developed was evaluated by the same methodology described. At the time of identifying the major contaminating compounds, pure pine cellulose was used as control cellulose.

To carry out the extractions, 1 g of cellulose was weighed in a previously tared Erlenmeyer flask. Then 100 mL of the extraction solvent (hexane, acetone, ethanol and water) was added and shaken for 4 hours at room temperature. Next, an aliquot of 1 mL was taken from the extractions carried out in water, ethanol and acetone, and filtered through a 0.45 $\mu$m nylon filters for injection into UPLC in ESI+ and ESI- mode. For the extraction carried out in hexane, the sample was evaporated and then reconstituted in the mobile phase ($H_2O$/MeOH).

A UPLC Waters Acquity I-Class liquid chromatograph with Q-TOF mass detector (flight time) EQ0182/ITN was used. The UPLC system was equipped with a Cortecs UPLC C18 1.5 $\mu$m (2.1 x 100 mm) column. For the ESI+ mode, $H_2O$/MeOH (0.1% formic acid) with a flow rate of 0.3ml/min was used as a mobile phase. For the ESI- mode, $H_2O$/MeOH (0.1% ammonium acid) was used at a flow rate of 0.3 ml/min.

On the other hand, for GC-TOF analysis, the samples were preconcentrated 100 times with Nitrogen (e.g., 50mL to 500$\mu$L). Again, they were filtered and injected. For GC analyses, Water7890A Gas chromatograph with mass detector TOP GCT Premier EQ04/ITN, the chromatographic method is as follows: injection volume 100 $\mu$l, carrier gas: hydrogen at 2mL/min; injections: split (1: 10) 250 °C, oven 40°C 3min;5°C/min up to 100 C, 10°C/min up to 280 °C (3 min), detection: scan 50-650 m/z and ESI+.

The extracts obtained following the previously mentioned protocols were analyzed by GC-TOF. The full-spectrum acquisition data were treated using an automated processing method, which consisted of obtaining between 2 and 5 nw-XICs (mass window 0.02 Da). The different compounds were identified by GC-TOF according to the NIST library provided by the equipment, following as acceptance criteria a mass error equal to or less than 3 mDa and a match equal to or greater than 700. The compounds identified in the extracts were taken from hexane, acetone and ethanol. The extracts obtained in the different solvents (except hexane, which was evaporated and replaced in methanol/water 1: 1v:v) were analyzed by UPLC-TOF

Volatile compounds identification: To identify the main volatile compounds, present in the sample, the material was weighed directly (0.5g) in a glass vial and then extracted with SPME (50/30 $\mu$m DVB/CAR/PDMS fiber). The chromatographic method was as follows: Water7890A Gas chromatograph with mass detector TOP GCT Premier EQ04/ITN, the chromatographic method is as follows: injection volume 1 mL, carrier gas: hydrogen at 2mL/min; injections: split (1: 10) 250 °C, oven 40°C 3min;5°C/min up to 100 C, 10°C/min up to 280 °C (3 min), detection: scan 50-650 m/z and EI.

Heavy metals analysis and quantification: For heavy metal extraction, cellulose sample (0.5 g) was taken in digestion tube and then 15 mL of a mix of $HNO_3$, $H_2SO_4$ and $HClO_4$ (5:1:1, v:v:v) was added to the sample. After an overnight stay, the tubes were put in digestion block present in the fuming hood and heated at 80 °C and then gradually increased the temperature up to 160 °C. After completion of digestion, the samples were cooled down, filtered and the final volume of 50 mL was made with distilled deionized water. The concentrations of metals were determined using atomic absorption spectrophotometer (AAS, Perkin-Elmer, A700) at University of Alicante (Spain). Each sample was analyzed in triplicate and mean value was taken for further interpretation.

Results: The identified compounds with the most significant levels by a semi-quantitative analysis was carried out by UPLC-TOF were: Cosmetics (Hexamethylene diisocyanate (103.3 ppm); Hexadecanamide (379.45 ppm); N-tetradecanamide (478.09 ppm); Oleamide (295.21 ppm); Adhesives ( Bis-(2-ethylhexyladipate) (182.80) (adhesive); Packaging (Dinonyl phthalate (DNIP) (201.56 ppm); Irganox 1081 (112 ppm); N,N-Diethyldecamide (650,52 ppm); Irganox 2246 (756.2 ppm) ); Food additives (Ascorbic acid (124.2 ppm); Stearic acid (451.1 ppm); Oleic acid (549.21 ppm); Palmitic acid (398.6 ppm); Catechin hydrate (174.32 ppm)).

The extracts obtained following the previously mentioned protocols were analysed by GC-TOF. More than 100 contaminants were detected, the origin of most of them comes from the cosmetic, food, packaging, pharmaceuticals, medicines, and detergent sectors.

The contaminating compound in greater proportion are nonadecane, and ethyl oleate, 4-hydroxy-2-pentanone, from the cosmetic industry.

Among the most relevant volatile compounds, those which are present in the sample in greater concentration, benzaldehyde, and 2-pentyl-furan, coming from the first two of the food industry while octanal can be found in certain cosmetic products.

Finally, regarding heavy metals present in the samples analyzed, Pb (67 ppm), Cu (35 ppm), Zn (110 ppm) and Ni (9 ppm) have been identified.

Example 3: Decontamination process following the process of the invention (named herein method 2)

The starting material was sample 1. The pollutant compounds found in greater proportion in this waste stream are phenolic compounds, amides, organic acids, some aromatic compounds, and fatty acids.

The alkaline treatment of the present invention is a chemical treatment in which the fibers are immersed in a known concentration of aqueous sodium hydroxide (NaOH) for a given temperature and a period of time in the presence of a chelating agent diethylenetriaminepentaacetic acid (DTPA). The pretreatment is carried out in 20 L reactor where is fill with a total volume of 17 L with a solid content of 5 % (w/w) with NaOH at 1 % (w/w) and 0.3 % (w/w) of DTPA. The reactor is maintained at 85 °C and 60 rpm for 2 hours. The NaOH and DTPA solution promote the removal of pollutants. Afterwards, the cellulose is washed four times with clean water. For that, the cellulose once treated with NaOH and DTPA, is filtered with a mesh to save the solid cellulose and discard the liquid. The cellulose retained was washed in a vessel with agitation. Once washed, the cellulose was filtered again to discard the water. This process was done 4 times. The washed cellulose was stored at 8 °C till it was used to perform the hydrolysis step.

Comparative example (method 1)

The alkaline treatment of Example 3 was carried out in the following more aggressive conditions: NaOH 3%/ DTPA 0.3%/ $H_2O_2$ 4%, $Na_2SiO_2$ 2.5%, at 90 °C; 1.5 atm of pressure and 2 h of treatment.

Example 4: Evaluation of decontamination degree

The cellulose treated with the methods defined, has been analyzed to determinate the pollutants removed and these results has been compared with pollutant present in non-treated cellulose. The analysis methods used UPLC-TOF and GC-TOF to determinate non-volatile and volatile compounds are as defined above.

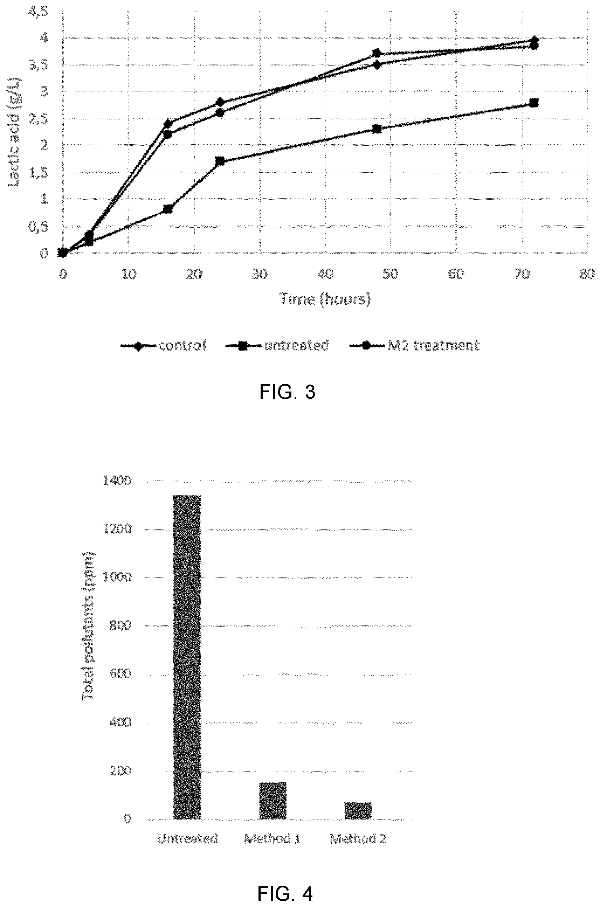

The results obtained in the analysis by UPLC-TOF are summarized in FIG. 4. The figure shows the total pollutants present in the non-treated cellulose and the pollutants presents in the cellulose treated with the method of the present invention. The results show that the pretreatment was effective to remove the pollutants present in the raw cellulose. In the semi-quantitative analyses carried out by UPLC-TOF, comparative method 1 showed an efficiency of 88.8%, while method 2 according to the invention reached a value higher than 95%.

In the analysis carried out by GC-TOF, 90% of the compounds were removed and analyzed for both methods. In addition, 80% of the volatile compounds identified in the contaminated cellulose samples were eliminated.

Finally, with respect to the levels of heavy metals, the Pb content was reduced by 85%, the Cu content by 74 and the Zn and Ni contents by 75 and 56%, respectively, for cellulose treated with method 2 (process of the present invention). The results are described in detail below.

Table 1 shows the decontamination percentages of the main pollutants identified by UPLC-TOF in ESI+ (DCS: Decontaminated Cellulosic Stream).

| UPLC-TOF Pollutant Analysis | | | | | |
|---|---|---|---|---|---|
| ESI + | | | | | |
| | | DECONTAMINATION | | | |
| Compound | DCS | Method 1 (comparative example) | | Method 2 (example of the invention) | |
| | ppm | ppm | % decontamination | ppm | % decontamination |
| 2,4-diaminoanisol | 0.99 | 0 | 100 | 0 | 100 |
| 2,4-dimethylaniline | 0.64 | 0.13 | 80 | 0 | 100 |
| 2,6-diaminotoluene | 4.6 | 0 | 100 | 0 | 100 |

(continued)

| UPLC-TOF Pollutant Analysis | | | | | |
|---|---|---|---|---|---|
| ESI + | | | | | |
| Compound | DECONTAMINATION | | | | |
| | DCS | Method 1 (comparative example) | | Method 2 (example of the invention) | |
| | ppm | ppm | % decontamination | ppm | % decontamination |
| 3,3-HDI derivatized | 103.3 | 0 | 100 | 0 | 100 |
| 3,5-ditertbutil-4-hidroxiacetofenona | 5.1 | 0 | 100 | 1.03 | 80 |
| Suberic acid | 4.24 | 0 | 100 | 0 | 100 |
| Benzyilbutilftalate (BBP) | 0.15 | 0.03 | 81 | 0.13 | 13 |
| Bis-(2-ethylhexyl)fumarate | 56.26 | 0 | 100 | 5.06 | 91 |
| Bis-(2- ethylhexyl )adipate | 182.80 | 0 | 100 | 0 | 100 |
| Diisodecil ftalate | 63.71 | 0 | 100 | 0 | 100 |
| Dinonil ftalate (DNIP) | 201.58 | 0 | 100 | 0 | 100 |
| Ethyl-3-(3,5-ditertbutil-4-hydroxyphenyl)propanoate | 6.02 | 0 | 100 | 0 | 100 |
| Ethylvainillin | 10.6 | 0 | 100 | 0 | 100 |
| Irganox 1098 | 53.06 | 46.2 | 13 | 0 | 100 |
| Methyl yellow | 10.34 | 0 | 100 | 0 | 100 |
| n-tetradecanamide | 478.09 | 62.1 | 87 | 0 | 100 |
| o-dianisidine | 12.06 | 0 | 100 | 0 | 100 |
| o-toluidine | 0.15 | 0 | 100 | 0 | 100 |
| Span 60 | 0.12 | 0 | 100 | 0 | 100 |
| sudan black 2 | 1.16 | 0 | 100 | 0 | 100 |
| Sudan Blue II | 7.42 | 0 | 100 | 6.7 | 10 |
| Sudan II | 46.66 | 34.52 | 26 | 0 | 100 |
| Tinuvin 328 | 0.14 | 0.06 | 53 | 0.07 | 49 |
| Tinuvin 770 | 38.51 | 1.53 | 96 | 1.54 | 96 |
| Uvinul 330 | 0.12 | 0 | 100 | 0.04 | 63 |

Table 2. Efficiency of decontamination methods using UPLC-TOF in ESI- mode. (DCS: Decontaminated Cellulosic Stream).

| UPLC-TOF Pollutant Analysis | | | | | |
|---|---|---|---|---|---|
| ESI - | | | | | |
| Compound | DECONTAMINATION | | | | |
| | DCS | Method 1 (comparative example) | | Method 2 (example of the invention) | |
| | ppm | ppm | % decontamination | ppm | % decontamination |
| 3,5-ditertbutil-4-hydroxibenzaldehide | 0.21 | 0 | 100 | 0 | 100 |

(continued)

| UPLC-TOF Pollutant Analysis | | | | | | |
|---|---|---|---|---|---|---|
| ESI - | | | | | | |
| Compound | | DECONTAMINATION | | | | |
| | | DCS | Method 1 (comparative example) | | Method 2 (example of the invention) | |
| | | ppm | ppm | % decontamination | ppm | % decontamination |
| Estearic acid. | | 451.1 | 0 | 100 | 0 | 100 |
| Carvacrol | | 2.65 | 2.64 | 0 | 1.77 | 33 |
| Methyl 3(3,5-ditertbutil-4-hydroxyphenyl) propia nate | | 0.87 | 0.86 | 0 | 0.86 | 0 |

The results derived from methods of decontamination of cellulose recovered from domestic waste water treatment plants by semi-quantification of contaminants by UPLC-TOF show a high effectiveness. The decontamination was higher than 95%.

Most of the compounds were effectively removed from the cellulose fibers. Some amides still persisted after decontamination of the samples, probably due to their high concentration, although the concentrations of these compounds decreased drastically. Some strongly hydrophobic compounds, such as methyl 3(3,5-ditertbutyl-4-hydroxyphenyl) propionate presented a very low decontamination rate, although their concentration in the sample barely reached 1 ppm.

After the analysis carried out by GC-TOF, where the presence/absence of the contaminating compounds and their relative abundance were detected, it was confirmed that the majority of the contaminating compounds were removed from the cellulose fibers, delimiting the detection of a few compounds, and also detecting levels much lower than those detected in the samples of the contaminated cellulose.

Table 3. Presence and relative abundance of the main pollutants in cellulose recovered from waste water treatment plant. GC-TOF analysis of samples extracted by hexane (ND: non-detected).

| Hexane Extract | | | | |
|---|---|---|---|---|
| Compound | Formula | Cellulose recovered | Method 1 (comparative example) | Method 2 (Example of the invention) |
| Cyclodecane | $C_{12}H_{24}$ | + | ND | ND |
| BHT | $C_{15}H_{24}O$ | + | + | + |
| Lauric Acid | $C_{12}H_{24}O_2$ | + | ND | ND |
| Lauric acid ethyl ester | $C_{14}H_{28}O_2$ | + | ND | ND |
| Hexadecane | $C_{16}H_{34}$ | + | ND | ND |
| 1-tetradecanol | $C_{14}H_{30}O$ | + | ND | ND |
| Myristic Acid | $C_{14}H_{28}O_2$ | + | ND | ND |
| ethyl myristate | $C_{16}H_{32}O_2$ | + | ND | ND |
| Octadecanoic | $C_{18}H_{39}$ | + | ND | ND |
| Nonadecane | $C_{19}H_{40}$ | ++ | ND | ND |
| Hexadecanoic Acid | $C_{16}H_{30}O_2$ | + | ND | ND |
| Eicosane | $C_{20}H_{42}$ | + | ND | ND |

(continued)

| Hexane Extract | | | | |
|---|---|---|---|---|
| Compound | Formula | Cellulose recovered | Method 1 (comparative example) | Method 2 (Example of the invention) |
| Cyclooctasulfide | $S_8$ | + | ND | ND |
| Heneicosane | $C_{21}H_{44}$ | + | ND | ND |
| Oleic acid | $C_{18}H_{34}O_2$ | +++ | ND | + |
| ethyl oleate | $C_{20}H_{38}O_2$ | ++ | ND | ND |
| Trico sane | $C_{23}H_{48}$ | + | ND | ND |
| Tetracosane | $C_{24}H_{50}$ | + | ND | ND |
| N-butyl benzenesulfamide | $C_{10}H_{15}NO_2S$ | + | + | ND |

Table 4. Presence/absence of the main pollutants in cellulose recovered from waste water treatment plant. GC-TOF analysis of samples extracted by ethanol (ND: non-detected).

| Ethanol Extract | | | | |
|---|---|---|---|---|
| Compound | Formula | Cellulose recovered | Method 1 (comparative example) | Method 2 (example of the invention) |
| N,N-dimethylacetamide | $C_4H_9NO$ | + | + | ND |
| Dodecanoic Acid | $C_{12}H_{24}O_2$ | + | ND | ND |
| Armeen DM 14D | $C_{16}H_{35}N$ | + | ND | ND |
| Tetradecanoic Acid | $C_{14}H_{28}O_2$ | + | ND | ND |
| N-butylbencenosulfamide | $C_{10}H_{15}NO_2S$ | ++ | + | + |
| Z-11- Hexadecanoic Acid | $C_{16}H_{30}O_2$ | + | ND | ND |
| Hexadecanoic acid | $C_{16}H_{32}O_2$ | ++ | ND | ND |
| Cyclooctasulfide | $S_8$ | + | ND | ND |
| Oleic Acid | $C_{18}H_{34}O_2$ | +++ | ND | + |

Table 5. Presence/absence of the main pollutants in cellulose recovered from waste water treatment plant. GC-TOF analysis of samples extracted by acetone (ND: non-detected).

| Acetone Extract | | | | |
|---|---|---|---|---|
| Compound | Formula | Cellulose recovered | Method 1 (comparative example) | Method 2 (example of the invention) |
| Toluene | $C_7H_8$ | + | ND | ND |
| Propane 2,2-diethoxy | $C_7H_{16}O_2$ | + | ND | ND |
| 4-hydroxy-2-pentanone- | $C_5H_{10}O_2$ | +++ | ND | ND |

(continued)

| Acetone Extract | | | | |
|---|---|---|---|---|
| Compound | Formula | Cellulose recovered | Method 1 (comparative example) | Method 2 (example of the invention) |
| Furan, 1-penthyl | $C_9H_{14}O$ | + | ND | ND |
| Dodecanal | $C_{12}H_{24}O_2$ | + | ND | ND |
| 1-dodecanol, methyl ether | $C_{13}H_{20}O$ | + | ND | ND |
| N,N-dimethyl-1-dodecanamide | $C_{14}H_{31}N$ | + | ND | ND |
| Dodecanoic Acid | $C_{12}H_{24}O_2$ | + | ND | ND |
| Armeen DM 14D | $C_{16}H_{35}N$ | + | ND | ND |
| octadecanoic Acid | $C_{14}H_{28}O_2$ | + | ND | ND |
| octadecane | $C_{18}H_{38}$ | + | ND | ND |
| 7-z-hexadecanoic acid | $C_{16}H_{30}O_2$ | + | ND | ND |
| palmitic acid | $C_{16}H_{32}O_2$ | +++ | + | ND |
| ciclooctasulfide | $S_8$ | + | ND | ND |
| Heneicosane | $C_{21}H_{44}$ | + | ND | ND |
| Oleic acid | $C_{18}H_{34}O_2$ | ++++ | ND | + |
| Tricosane | $C_{23}H_{48}$ | + | ND | ND |

Table 6: Volatile compounds present in cellulose before and after decontamination process (nd: non detected)

| Volatile compounds present in the samples before and after decontamination methods M1 (comparative example using stronger conditions as in the prior art) and M2 (example according to the invention) are collected in this Table. As it can be seen both methods were effective in the elimination of the majority of contaminants, representing a value lower than 10% of the original sample. | | | | |
|---|---|---|---|---|
| Volatile compounds | | | | |
| Compound | Molecular formula | Cellulose recovered | Method 1 (comparative example) | Method 2 (example of the invention) |
| Isopentanal | $C_5H_{10}O$ | ++ | ND | ND |
| Azetidine | $C_3H_7N$ | + | ND | ND |
| Hexanal | $C_6H_{12}O$ | ++ | ND | ND |
| Methyl pirazine | $C_5H_6N_2$ | + | ND | ND |
| Furfural | $C_5H_4O_2$ | + | ND | ND |
| Furfuryl alcohol | $C_5H_6O_2$ | + | ND | ND |
| Heptanal | $C_7H_{12}O$ | ++ | ND | ND |
| Benzaldehyde | $C_7H_6O$ | +++ | + | + |
| 2-penthyl furan | $C_9H_{14}O$ | +++ | ND | ND |

(continued)

| Volatile compounds present in the samples before and after decontamination methods M1 (comparative example using stronger conditions as in the prior art) and M2 (example according to the invention) are collected in this Table. As it can be seen both methods were effective in the elimination of the majority of contaminants, representing a value lower than 10% of the original sample. | | | | |
|---|---|---|---|---|
| Volatile compounds | | | | |
| Compound | Molecular formula | Cellulose recovered | Method 1 (comparative example) | Method 2 (example of the invention) |
| Octanal | $C_8H_{16}O$ | +++ | + | + |
| Pyrrole-2-carboxaldehyde | $C_5H_5NO$ | + | ND | ND |
| y-caprolactrone | $C_6H_{10}O_2$ | + | ND | ND |
| 2-e-octenal | $C_8H_{14}O$ | + | ND | ND |
| 2-cloro octane | $C_8H_{17}Cl$ | + | ND | ND |
| 2-nonanone | $C_9H_{18}O$ | ++ | + | ND |
| Noanal | $C_9H_{18}O$ | + | ND | ND |
| Phenylethyl alcohol | $C_8H_{10}O$ | +++ | ND | ND |
| $\alpha$-isophoran | $C_9H_{14}O$ | + | ND | ND |
| Methyl nonyl ether | $C_{10}H_{22}O$ | + | ND | ND |
| 2-decanone | $C_{10}H_{20}O$ | + | ND | ND |
| Dodecane | $C_{18}H_{26}$ | + | ND | ND |
| Decanal | $C_{10}H_{20}O$ | + | ND | ND |
| 2-e-decenal | $C_{10}H_{18}O$ | + | + | + |
| tetradecane | $C_{14}H_{30}$ | + | ND | ND |
| Dodecanal | $C_{12}H_{24}O$ | + | + | ND |
| Valencene | $C_{15}H_{24}$ | + | ND | ND |
| Galoxide | $C_{15}H_{10}O$ | + | + | ND |
| 1-dodecanol | $C_{12}H_{26}O$ | ++ | ND | ND |
| Pentadecane | $C_{15}H_{32}$ | + | ND | ND |
| Sulfur | $S_6$ | + | ND | ND |
| Dihydro actinidiolide | $C_{11}H_{16}O_2$ | + | ND | ND |
| Hexadecane | $C_{16}H_{34}$ | + | ND | ND |
| Octadecane | $C_{18}H_{38}$ | ++ | ND | ND |
| 2-methoxynaftalene | $C_{18}H_{26}O$ | + | + | + |
| Sulfur mol. | $S_8$ | + | ND | ND |

The data indicate that there was a reduction in most of the volatile contaminants representing a value of less than 10% of the original sample.

Table 7. Efficiency of decontamination methods for the removal of heavy metals.

| HEAVY METAL | Cellulose recovered initial value | Method 2 of the present invention | Decontamination % |
|---|---|---|---|
| Pb (ppm) | 67 | 10 | 85 |
| Cu (ppm) | 35 | 9 | 74 |
| Zn (ppm) | 110 | 28 | 75 |
| Ni (ppm) | 9 | 4 | 56 |
| | | | |

A high effectiveness in terms of heavy metal removal (a concentration of 0.3% DTPA as a chelating compound was added) is obtained, eliminating 72.5%. These results are shown in Table 7.

Example 4: Saccharification process

The cellulose (sample 1) decontaminated according to the method of the present invention was hydrolyzed into fermentable sugars by β-glucosidases (1000 U/g), with an enzyme dosage of 0.05 mL/g dry solid. The hydrolysis conditions are 48h at 45°C and with stirring at 150 rpm.

Saccharification test was carried out in semibatch mode in 30 L-bioreactor with 10 L of working volume: 12 g/L concentration of the dried sample were homogenously distributed in 3 feds: at 0, 6 and 24 h. To evaluate the amount of glucose, samples were taken at 0, 6, 24, 30 and 48 hours. In order to quantify the total reducing sugar, DNS method was followed (See W.L. Marsden et al., "Evaluation of the DNS method for analyzing lignocellulosic hydrolysates"; journal of Chemical Technology and Biotechnology, 1982, vol. 32, pp. 1016-1022). The reagent is a solution formed by the following compounds: 3, 5-Dinitrosalicylic acid (2-hydroxy-3,5-dinitrobenzoic acid), which acts as an oxidant, Rochelle salt (sodium-potassium tartrate), which prevents the dissolution of oxygen in the reagent, and sodium hydroxide were added to provide the medium required for the redox reaction to occur.

Results: After carrying out the different tests, a hydrolysate containing 62.42 g/L of glucose was obtained. In this case, 41.55 g/L of glucose were released, corresponding with a 95% of the theoretical yield.

Example 5: Fermentation process

Once the effect of the decontamination processes on the enzymatic hydrolysis process had been analyzed, the possible toxicity of the cellulose treated in a fermentative process was evaluated in the bioconversion of the 2nd generation sugars obtained for the production of lactic acid. For carrying out this fermentation, the reference strain CECT 923 of *Pediococcus pentosaceus* was used.

Each flask (100 mL) was inoculated with 1 ml of the *Pediococcus pentosaceus* and incubated at 30 °C for 72 hours. A sample was taken immediately after adding the microorganism at 4, 16, 24, 48, and 72 hours.

Parameters such as cell viability and yield in the production of lactic acid were evaluated. With the collected samples, the concentration of lactic acid was analyzed by High-Efficiency Liquid Chromatography (HPLC). The system consists of solvent elements, an automatic sample injector and a UV detector programmed to detect lactic acid at 211 nm.

The HPLC system used a Rezex ROA-Organic acids H+ column (8%), 300*7.8 mm in diameter. 9 calibration points 10, 50, 100, 250, 500, 1000, 2500, 5000, 10000 ppm were prepared as the pattern. The stock solution was made of 1.19 ml of the lactic acid solution and 25 ml of water (10000 ppm). 10 μL were injected per sample, following the method designed by Doyon et al., 1991, with little modifications.

FIG. 3 shows the results obtained after analyzing the concentration of lactic acid produced by *P. pentosaceus*, using different extracts derived from enzymatically hydrolyzed cellulosic samples (control, untreated, and decontaminated by Method 2) containing 2nd generation sugars. As main results, it can be observed that lower yield was obtained from the non-decontaminated sample, while the yield significantly increased when the sample was subjected to Method 2 decontamination process. In fact, the hydrolysate derived from Method 2 decontamination method (example of the invention) achieved concentrations similar to those obtained with the control sample (pure cellulose).

FIG. 4 shows *Pediococcus pentosaceus* colonies after 48h of incubation at 30°C. Only the Petri dishes with 30 to 300 colonies were used to calculate CFU/mL (colony-forming unit per mL) and the represented by the log (CFU/mL) in FIG. 4.

*Pediococcus pentosaceus* could grow to a greater extend from Method 2 treated sample, while the viability of the microorganism was considerably lower when using the untreated sample.

**Citation List**

Patent Literature

- US2012083542A
- CA2071290AA
- US2002185447 A1
- CN106914225B
- CN102174602A

Non Patent Literature

- A. ilbazarri et al.; "Alkali treated foumanant tea waste as an efficient adsorbent for methylene blue adsorption from aqueous solution", Water Resour Ind, 2014, 6, 64-80

- Y. Zhou et al, "Removal of organic pollutants from aqueous solution using agricultural wastes: a review, Journal of Molecular liquids 2015, 212, 739-762

- L.E. Wise et al., Tech. Assoc. Papers 1946, vol. 29, p.210

- R.M. Rowell, "Distribution of acetyl groups in southern pine reacted with acetic anhydride" Wood Sic, vol. 15, pp.172-182

- W.L. Marsden et al., "Evaluation of the DNS method for analyzing lignocellulosic hydrolysates"; journal of Chemical Technology and Biotechnology, 1982, vol. 32, pp. 1016-1022

- https://www.lgcstandards-atcc.org/en/About/About_ATCC/Who_We_Are.aspx.

- D. Gopaliya et al., "Recent advances I itaconic acid production from microbial cell factories", Biocatalysis and Agricultura Biotechnology. 2021, vol. 36, 102130, pp1-14.

- R. Reshmy et al., "Bioplastic production from renewable lignocellulosic feedstrocks; a review; Rev, Environ. Sic. Biotechnol, 2021, vol. 20, pp. 167-187.

- J. Cheng et al., "Achievements and Perspectives in 1,4-Butanediol Production from Engineered Microoganisms", 2021, J. Agric. Food Chem, 69, 10480-10485.

**Claims**

1. A process of decontamination of a recovered cellulosic waste stream, which comprises:

   a) submitting the recovered cellulosic waste stream to an alkaline treatment which comprises the following steps:

   a1) combining the recovered cellulosic waste stream with diethylene triamine pentaacetate (DTPA) and a base to yield an alkaline medium wherein the ratio cellulose:base is in a range from 8:1 to 3:1 weight %;
   a2) heating up the reaction mixture to a temperature in a range from 75 to 90 °C and atmospheric pressure for at least 1 hour; and
   a3) separating a decontaminated cellulosic stream.

2. The process according to claim 1, wherein the recovered cellulosic waste stream comes from a waste stream selected from the group consisting of a sewage sludge, a municipal sewage water, and domestic wastewater.

3. The process according to any of the claims 1-2, wherein the recovered cellulosic waste stream comprises hollocellulose in an amount comprised in the range from 80% to 90% by weight with respect to the recovered cellulosic waste stream.

4. The process according to any of the claims 1-3, wherein the recovered cellulosic waste stream comprises α-cellulose in an amount comprised in the range from 70% to 95% by weight with respect the hollocellulose; and hemicellulose in an amount comprised in the range from 5% to 15% by weight with respect to the hollocelulose.

5. The process according to any of the claims 1-4, wherein the recovered cellulosic waste stream is in the form of fibers which have a length comprised in a range from 800 to 1100 μm; and a width comprised in a range from 10 to 25 μm.

6. The process according to any of the claims 1-5, wherein the ratio cellulose:base in the alkaline treatment is comprised in a range from 6:1 to 4:1 weight %.

7. The process according to any of the claims 1-6, wherein the base in the alkaline treatment is sodium hydroxide.

8. The process according to any of the claims 1-7, wherein the amount of diethylenetriamine pentaacetate in the alkaline treatment is comprised in a range from 0.1 to 1 %w/w.

9. The process according to any of the claim 1-8, wherein the temperature of the alkaline treatment is comprised between 80-85 °C.

10. The process according to any of the claims 1-9, wherein the separation of the decontaminated cellulosic stream from the alkaline solution is by filtration and/or decantation.

11. The process according to any of the claims 1-10, in which the alkaline treatment is carried out in the absence of any oxidizing agent.

12. A process of decontamination of a recovered cellulosic waste stream and subsequent transformation of the decontaminated cellulosic waste stream into a hydrolysate comprising fermentable sugars, comprising the following steps:

   a) carrying out the process according to any of the claims 1-11,
   b) submitting the decontaminated cellulosic stream of step a3) to a saccharification process to obtain a hydrolysate which comprises fermentable sugars.

13. The process according to any of the claim 12, wherein the hydrolysis of the treated cellulose of step a3) into fermentable sugars is carried out by a consortium of enzymes selected from the group consisting of: β-glucosidases (600-1000 U/g) (Enzymatic cocktail 1); cellulases (100-200 U/g), xylanases (100-200 U/g), and β-glucosidases (300-600 U/g) (Enzymatic cocktail 2); and endo and exo cellulases (100-200 U/g) (Enzymatic cocktail 3).

14. The process according to claim 13, which is carried out at a temperature comprised in a range from 45 to 50 °C.

15. A process of decontamination of a recovered cellulosic waste stream and subsequent transformation of the decontaminated cellulosic stream into a compound selected from lactic acid, succinic acid, itaconic acid 1,4-butanediol, and polyhydroxyalkanoates, comprising the following steps: carrying out the process according to any of the claims 12-14, and then carrying out a step c) comprising submitting the hydrolysate which comprises fermentable sugars to a fermentation process in the presence of a microorganism capable of producing a compound selected from the group consisting of lactic acid, succinic acid, itaconic acid 1,4-butanediol, and polyhydroxyalkanoates at an appropriate temperature and an appropriate incubation time.

FIG. 1

FIG. 2

FIG. 3

FIG. 4

FIG. 5

Europäisches
Patentamt

European
Patent Office

Office européen
des brevets

**EUROPEAN SEARCH REPORT**

Application Number

EP 22 38 3286

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/060294 A1 (NAT UNIV SINGAPORE [SG]) 24 March 2022 (2022-03-24) <br> * page 1, lines 20-26 * <br> * page 2, lines 5-10 * <br> * page 2, line 24 – page 3, line 4 * <br> * page 4, lines 21-28 * <br> * page 5, lines 1-18,27 * <br> * page 6, lines 1-16,30,31 * <br> * page 7, lines 5-15 * <br> * page 8, lines 13,14 * <br> * page 9, lines 1-29 * <br> * examples; <br> pages 10-22; figure 5 * <br> ----- | 1-15 | INV. <br> C02F1/66 <br> C02F1/68 <br> C02F11/00 <br> C02F11/02 <br><br> ADD. <br> C02F103/28 <br> C02F3/34 <br> C12P7/56 <br> C12P7/46 <br> C12P7/625 <br> C08J11/04 <br> C13K1/02 <br> C12P19/14 <br> C08H8/00 |
| A | KO CHUN-HAN ET AL: "Impact of pretreatment methods on production of bioethanol and nanocrystalline cellulose", JOURNAL OF CLEANER PRODUCTION, ELSEVIER, AMSTERDAM, NL, vol. 254, 31 December 2019 (2019-12-31), XP086079695, ISSN: 0959-6526, DOI: 10.1016/J.JCLEPRO.2019.119914 [retrieved on 2019-12-31] <br> * abstract * <br> * page 2, right-hand column, lines 12-31 * <br> * page 6, right-hand column, paragraph 3.5. * <br> * page 7; figure 6 * <br> ----- | 1-15 | |
| | | | TECHNICAL FIELDS SEARCHED (IPC) <br><br> C02F <br> C12P <br> C09J <br> C08J <br> C13K <br> C08H |

The present search report has been drawn up for all claims

| Place of search | Date of completion of the search | Examiner |
|---|---|---|
| The Hague | 5 June 2023 | Vaz, Miguel |

## ANNEX TO THE EUROPEAN SEARCH REPORT
## ON EUROPEAN PATENT APPLICATION NO.

EP 22 38 3286

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2023

| Patent document cited in search report | Publication date | Patent family member(s) | Publication date |
|---|---|---|---|
| WO 2022060294 A1 | 24-03-2022 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 2071290 AA
- US 2002185447 A1
- CN 106914225 B
- US 2012083542 A
- CN 102174602 A

**Non-patent literature cited in the description**

- **A. ILBAZARRI et al.** Alkali treated foumanant tea waste as an efficient adsorbent for methylene blue adsorption from aqueous solution. *Water Resour Ind,* 2014, vol. 6, 64-80
- **Y. ZHOU et al.** Removal of organic pollutants from aqueous solution using agricultural wastes: a review. *Journal of Molecular liquids,* 2015, vol. 212, 739-762
- **J. CHENG et al.** Achievements and Perspectives in 1,4-Butanediol Production from Engineered Microoganisms. *J. Agric. Food Chem,* 2021, vol. 69, 10480-10485
- **D. GOPALIYA et al.** Recent advances in itaconic acid production from microbial cell factories. *Biocatalysis and Agricultura Biotechnology.,* 2021, vol. 36 (102130), 1-14
- **R. RESHMY et al.** Bioplastic production from renewable lignocellulosic feedstrocks; a review. *Rev, Environ. Sic. Biotechnol,* 2021, vol. 20, 167-187
- **L.E. WISE et al.** *Tech. Assoc. Papers,* 1946, vol. 29, 210
- **R.M. ROWELL.** Distribution of acetyl groups in southern pine reacted with acetic anhydride. *Wood Sic,* vol. 15, 172-182
- **W.L. MARSDEN et al.** Evaluation of the DNS method for analyzing lignocellulosic hydrolysates. *journal of Chemical Technology and Biotechnology,* 1982, vol. 32, 1016-1022
- **D. GOPALIYA et al.** Recent advances I itaconic acid production from microbial cell factories. *Biocatalysis and Agricultura Biotechnology.,* 2021, vol. 36 (102130), 1-14